# EUROPÄISCHE PATENTSCHRIFT

(11) **EP 0 392 199 B1**
(45) Veröffentlichungstag und Bekanntmachung des Hinweises auf die Patenterteilung: **23.06.1993**
(21) Anmeldenummer: 90104763.9
(22) Anmeldetag: 14.03.1990
(51) Int. Cl.: A61M 5/00, A61M 5/165, A61M 25/00

(54) **Port zur Einspritzung von Medikamenten**
Port for injection of medicaments
Port pour injection de médicaments

(30) Priorität: 11.04.1989 DE 8904527 U
(43) Veröffentlichungstag der Anmeldung: 17.10.1990
(73) Patentinhaber: B. Braun Melsungen AG, 34209 Melsungen (DE)
(72) Erfinder: Brethauer, Ullrich, D-3501 Körle (DE)
(74) Vertreter: Selting, Günther, Dipl.-Ing.

(56) Entgegenhaltungen:
- DE-U- 8 434 177
- US-A- 4 464 178
- US-A- 4 704 103

## Beschreibung

Die Erfindung betrifft einen Port zur Einspritzung von Medikamenten durch einen implantierten Katheter, bestehend aus einer Kapsel, die aus einem Boden, einer einen Hohlraum umgebenden Umfangswand und einer den Hohlraum verschließenden durchstechbaren Oberwand gebildet ist und die in der Umfangswand eine Auslaßöffnung aufweist, die innen durch einen gegen den Querschnitt der Auslaßöffnung in der Umfangswand angesetzten Filterkörper abgeschirmt ist und an die ein Ende des Katheters angeschlossen ist.

Derartige Ports, die auch als "Zuspritzteil" bezeichnet werden, sind bekannt (DE 33 09 788) und sie werden im allgemeinen mit dem Katheter zusammen implantiert. Die Kapsel hat die Form einer runden flachen Schale, deren Hohlraum von der aus einer elastischen Membran gebildeten Oberwand dicht verschlossen ist. Der obere Schalenrand ist so ausgebildet, daß eine möglichst große elastische Membranfläche als Einstichfläche zur Verfügung steht. In der Ebene des Bodens weist die Kapsel einen radial abstehenden ringförmigen Flansch auf, der mit Löchern zum Festnähen der Kapsel an der Faszie versehen ist. Die Auslaßöffnung in der Umfangswand der Kapsel steht bei dem erwähnten bekannten Port mit einem radialen Anschlußstutzen in Verbindung, auf den das Katheterende aufsteckbar ist. Eine verbesserte Verbindung zwischen Port und Katheter ergibt sich durch Einschieben des Katheters in einen Anschlußstutzen der Kapsel und Festklemmen desselben mittels eines hülsenförmigen elastomeren Klemmstückes, das von einem mit dem Anschlußstutzen verbindbaren Druckstück radial deformierbar ist und den Katheter festspannt (DE 36 28 337).

Im praktischen Gebrauch kommt es immer wieder vor, daß die durchstechbare Oberwand (Portmembran) nicht mit speziell für dieses Einsatzgebiet vorgesehenen Portkanülen perforiert wird, sondern daß von dem Anwender herkömmliche Kanülen mit ungünstiger Dicke und Schliffgeometrie eingesetzt werden. Dies führt dazu, daß feine Materialspäne aus der elastomeren Oberwand ausgestanzt werden, die die Auslaßöffnung in der Umfangswand der Kapsel bzw. den Katheter selbst verstopfen können oder die in die Blutbahn eingeschleppt werden. Dies soll bei einer subkutanen Eingabevorrichtung gemäß DE 36 41 107 durch einen porösen Schaumstoffstöpsel verhindert werden, der von einem Metallsieb abgedeckt oberhalb der Auslaßöffnung den Hohlraumquerschnitt der Kapsel überspannend und zu ihrem Boden parallel verlaufend angeordnet ist. Diese Ausbildung ist nicht dauerhaft zuverlässig, weil sich der plattenförmige Schaumstoffstöpsel, der nur am Rand in dem Hohlraum der Kapsel gehalten ist, unter dem mehrfachen Andruck der Kanülenspitze gegen das Metallsieb in der Kapsel lockern und dadurch am Rande undicht werden kann.

US-A-4 464 178 beschreibt ein Filter, das gegen den Querschnitt der Auslaßöffnung in der Umfangswand angesetzt ist und aus einem Titandraht-Maschennetz besteht, dessen Rand zwischen zwei Platten eingelagert ist, die beide aus Silikongummi bestehen. Ein zentraler Durchbruch in den beiden Platten ist von dem Maschennetz überspannt. Ein Filterkörper in Form eines Draht-Maschennetzes hat zwangläufig verhältnismäßig große Poren mit der Folge, daß Feinstpartikel nicht zurückgehalten werden, sondern in den Katheter gelangen können. Ferner ist es nachteilig, daß der Plattenaufbau vom Inneren des Hohlraumes der Kapsel her in eine Erweiterung der Auslaßöffnung festsitzend eingepaßt werden muß, wobei eine Sichtkontrolle nicht möglich ist. Jede durch Maßabänderungen und/oder Montagefehler erzeugte Randdeformation des flexiblen Plattenaufbaus kann zur Bildung von Durchlässen führen, die noch größer als die Maschenweite sind und eine weitere Verschlechterung der Filterfunktion bewirken.

Der Erfindung liegt die Aufgabe zugrunde, einen Port der eingangs erwähnten Art so zu verbessern, daß sein Filter hochwirksam und über lange Zeiträume voll funktionsfähig ist.

Diese Aufgabe wird durch die Merkmale der Ansprüche 1 und 2 gelöst.

Ein Filterkörper aus Sintermaterial mit einer Porengröße von beispielsweise 20 µm bewirkt, daß nach Perforation mit einer stanzenden Kanüle abgehobelte Späne des Elastomermaterials der Oberwand an dem Filterkörper zurückgehalten werden. Die Stanzteile sind aufgrund der Filterfeinheit in dem Hohlraum der Kapsel wie in einem Käfig gefangen und können nicht durch die englumige Auslaßöffnung des Ports gelangen. Diese wird zuverlässig offengehalten und auch der Katheter kann nicht verstopfen oder Späne in den Blutkreislauf des Patienten weitertragen. Unabhängig von der Häufigkeit der Punktion der durchstechbaren Oberwand der Kapsel ist die Sicherheit beim Zuspritzen durch den Port garantiert, weil der Filterkörper in senkrechter Ebene neben der Bewegungsebene der Punktionskanüle liegt, so daß er mit der Spitze der Ranüle gar nicht in Berührung kommt. Da die Druckkraft der durch die Oberwand stechenden Kanülenspitze nie auf den Filterkörper wirkt, wird sein Sitz in bezug auf die Auslaßöffnung von der Kanülenspitze nicht beeinflußt.

Vorteilhafterweise besteht das Sintermaterial des Filterkörpers aus hochporösem rostfreiem Stahl oder aus anderen metallischen Werkstoffen, wie Nickel, Hastelloy C, B und X, Titan, Monel, Inconel, Incolog. Diese und andere Qualitäten werden dann eingesetzt, wenn Eisenbasislegierungen für die Korrosionsbeanspruchung nicht mehr ausreichen. So kann es bei dem vorgesehenen Einsatzgebiet in Verbindung mit einem salzsauren Präparat von Vorteil sein, den hochporösen Filterkörper aus Titan herzustellen. Je nach Anwendung sind Filterfeinheitsabstufungen im Bereich von 0,5 bis 200 µm vorteilhaft.

Ein weiterer Vorteil der Herstellung des Filterkörpers aus Sintermetall besteht darin, daß keine zusätzlichen Kontrastringe zur Röntgendarstellung in die Kapsel integriert werden müssen, weil der Filterkörper aus Metall einen optimalen Röntgenkontrast bietet.

Das Sintermaterial kann alternativ aus Kunststoff oder aus keramischem Material, zum Beispiel Glas, bestehen. In beiden Fällen sorgt der hochporöse Filterkörper für eine Sicherung der Auslaßöffnung des Ports durch Zurückhaltung von Fremdpartikeln im Hohlraum bei guter Durchströmbarkeit und Haltbarkeit.

Bei der Ausführungsform der Erfindung nach Anspruch 2 ist der Filterkörper als Scheibe aus hochporösem Sintermaterial ausgebildet, die in einer oben offenen Tasche der Umfangswand angeordnet ist, deren Innenwand eine Bohrung aufweist, die mit der Auslaßöffnung fluchtet. Die Scheibe läßt sich einfach und korrekt montieren. Sie wird in den Auslaufkanal integriert und schützt ihn gegen Verstopfung. Allerdings ist hierbei die Möglichkeit nicht ausgeschlossen, daß die Bohrung in der Innenwand der Tasche durch ausgestanzte Partikel zugesetzt wird.

Einen umfassenderen Schutz bietet der Filterkörper nach Anspruch 1, der als topf- oder ringförmiges Einsatzteil ausgebildet ist, dessen Umfangsform und Höhe dem Hohlraum der Kapsel angepaßt ist. Das topf- oder rinförmige Teil kann außen und/oder innen konisch bzw. zylindrisch sein. Die Außenform richtet sich nach dem Verlauf der Innenseite der Umfangswand, die den Hohlraum umgibt, während die Innenform Zweckmäßigkeitserwägungen unterliegt. Bevorzugt ist eine konische Innenfläche.

In vorteilhafter Ausgestaltung der Erfindung ist der der Oberwand benachbarte Rand des Einsatzteils als Schneidkante ausgebildet, die in die Oberwand eindringt. Auf diese Weise wird die Dichtigkeit zu der angrenzenden elastischen Membran verbessert, so daß auch keine Partikel unterhalb der Oberwand hinter den Filterkörper gelangen können. Ferner wird durch die Schneidkante ein festerer Preßsitz der an der Kapsel befestigten elastomeren Oberwand erreicht.

Bei einer besonders bevorzugten Ausführungsform der Erfindung liegt der Bodenteil des Einsatzteils gegen den Boden des Hohlraumes der Kapsel an. Dies ist besonders güngstig, weil das Sintermetall des Einsatzteils den Boden der Kunstotoffkapsel gegen perforierende Einstiche schützt, die sich bisher bei unsachgemäßen Einstichen mit überhöhtem Kraftaufwand ergeben haben. Der mit dem Ring einteilig geformte Bodenteil aus Sintermetall deckt den Boden des Kapselhohlraumes ab und schützt ihn, so daß er nicht mehr versehentlich perforiert werden kann. Die Zuverlässigkeit des Ports wird erhöht, weil keine Leckstellen für die in die Kapsel eingespritzte Medikamentendosis vorhanden sind. Ein Filterkörper mit Bodenteil als Durchstechschutz bedingt einen etwas höheren Hohlraum der Kapsel, was sich jedoch auf die Gesamthöhe der zu implantierenden Kapsel nicht auswirken muß, weil der durch den Bodenteil des Sintermetallringes geschützte Boden der Kapsel dünner als bisher ausgebildet sein kann.

In der Zeichnung sind Ausführungsbeispiele der Erfindung schematisch dargestellt. Es zeigt:
Fig. 1 eine explodierte Darstellung eines Ports mit Filterkörper im Querschnitt,
Fig. 2 eine Draufsicht auf eine Hälfte des Ports ohne Halterung und Oberwand,
Fig. 3 und 4 Querschnitt und Draufsicht des bei dem Beispiel der Figuren 1 und 2 verwendeten Filterkörpers,
Fig. 5 einen Querschnitt durch einen Port mit einer anderen Ausführungform eines Filterkörpers,
Fig. 6 und 7 Querschnitt und Draufsicht auf einen als Ring ohne Boden ausgebildeten Filterkörper,
Fig. 8 und 9 Querschnitt und Draufsicht auf eine andere Form eines Ringes ohne Boden und
Fig. 10 und 11 Querschnitt und Draufsicht auf einen Port, der mit einem scheibenförmigen Filterkörper ausgestattet ist.

Der Port 10 besteht aus einer Kapsel 11, die in Draufsicht kreisförmig ist. Die Kapsel 11 weist einen Boden 12, eine kreisförmige Umfangswand 13 und eine durchstechbare Oberwand 14 auf, die als dicke elastische Membran aus Silikonkautschuk gebildet ist. Der Boden 12, die Oberwand 14 und die Umfangswand 13 mit kreiszylindrischer Innenseite 20 umschließen einen Hohlraum 15, der mittels einer durch die Oberwand 14 gestochenen Kanüle mit einem flüssigen Medikament befüllbar ist. Die Oberwand 14 ist als kreisförmige profilierte Scheibe mit doppelt abgestuftem zylindrischen Außenumfang ausgebildet. Ein zentraler dicker Teil 14a ist von einer radial vorgezogenen, abgesetzten Ringschulter 14b umgeben und ein von der Unterfläche 14d axial vorstehender Rand 14c bildet ein Auflager, das in eine unterste Ringaussparung 16 kleinsten Durchmessers in der Innenfläche der Umfangswand 13 eingepaßt ist. Ein Haltering 18, der mit einem koaxialen Ansatz 18a in eine äußerste Ringaussparung 17 größten Durchmessers der Umfangswand 13 hineinragt, übergreift mit einem einwärts gebogenen Randflansch 18b die Ringschulter 14b der Oberwand 14 und drückt diese gegen ihren Sitz. Die Umfangswand 13 und der Haltering 18 sind miteinander verklebt oder verschweißt. Der Randflansch 18b des Halteringes 18 bildet einen wulstförmigen Kragen, der den Zentralteil 14a der Oberwand 14 umgibt und ihr Ertasten bei implantiertem Port durch die Haut hindurch erleichtert.

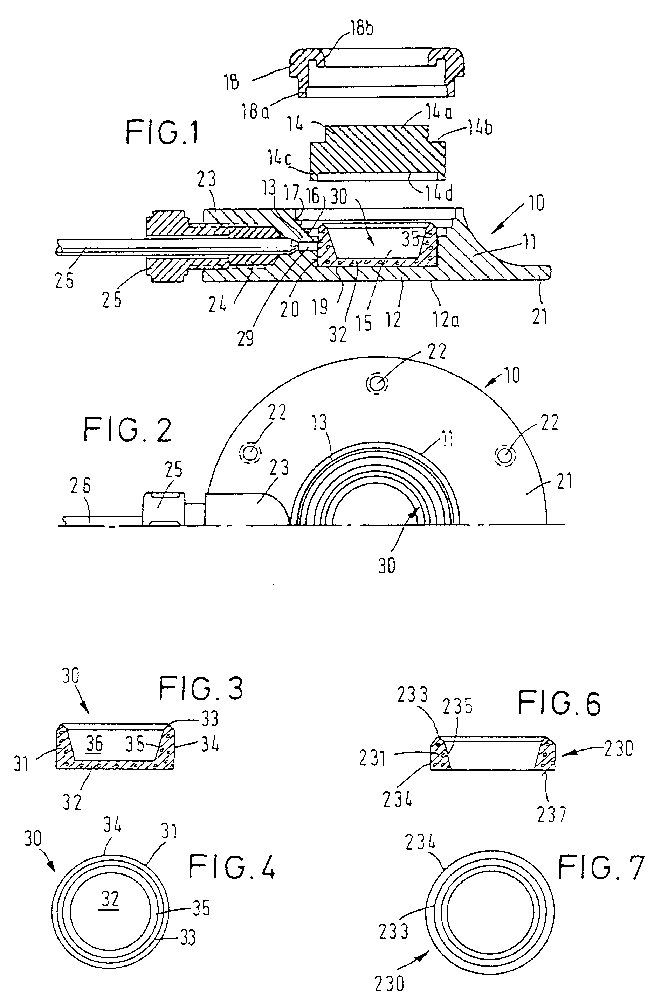

In der Ebene des Bodens 12 ist an die Kapsel 11 ein radial abstehender ringförmiger Flansch 21 angeformt, der mit Löchern 22 (Fig. 2) zum Festnähen des Ports 10 an der Faszie versehen ist. Die Unterfläche 12a des Bodens 12 und des Flansches 21 bilden eine glatte Fläche, die parallel zur ebenen, glatten Innenfläche 19 des Bodens 12 verläuft. Von dem Flansch 21 erhebt sich ein Anschlußstutzen 23 nach oben, der außen in die Umfangswand 13 der Kapsel 11 übergeht. An seinem freien Ende steht der Anschlußstutzen 23 nicht über den äußeren Rand des Flansches 21 vor. Der Anschlußstutzen 23 weist einen zylindrischen Klemmraum auf, in dem ein angepaßtes elastomeres Klemmstück 24 in Hülsenform untergebracht ist, gegen das axial die Stirnfläche eines Druckstückes 25 anpreßt, das mittels eines Innengewindes im Klemmraum und eines Außengewindes an dem Druckstück 25 axial verstellbar ist. Das Druckstück 25 und das Klemmstück 24 sind von einem koaxialen Längskanal durchsetzt, der dem Durchlaß eines Katheters 26 dient, der von dem sich radial verformenden Klemmstück 24 in dem Anschlußstutzen 23 festgespannt wird. Der Kanal in dem Klemmstück 24 und dem Druckstück 25 fluchtet mit einer Auslaßöffnung 29 in der Umfangswand 13 der Kapsel 11, welche auf der Innenseite 20 mündet und den Hohlraum 15 mit dem Katheter 26 verbindet.

In den Hohlraum 15 ist ein passender Filterkörper 30 eingefügt, der in den Figuren 3 und 4 im einzelnen dargestellt ist. Der Filterkörper 30 besteht aus einem Ring 31 aus hochporösem Sintermaterial, wie rostfreiem Stahl, Nickel, Titan oder ähnlichen geeigneten metallischen Werkstoffen und an sein eines Ende ist ein Bodenteil 32 aus dem gleichen Werkstoff angeformt. Der Ring 31 des Filterkörpers 30 hat eine kreiszylindrische Außenwand 34, deren Durchmesser so gewählt ist, daß er dem Durchmesser des Hohlraumes 15 exakt angepaßt ist. Die Innenwand 35 des Ringes 31 verläuft konisch und verjüngt sich gegen den Bodenteil 32. An dem freien äußeren Rand des Ringes 31 ist eine Schneidkante 33 ausgebildet, die vorzugsweise einen Flankenwinkel von 90° hat. Die Höhe des Filterkörpers 30 ist so vorgesehen, daß bei Abstützung des Bodenteils 32 auf der Innenfläche 19 des Bodens 12 der Kapsel 11 die Schneidkante 33 in die Unterseite 14d der elastomeren Oberwand 14 eindringt, so daß die offene Seite des Ringes 31 dicht und mit etwas Druck an der elastischen Oberwand 14 anliegt und diese einen festeren Preßsitz erhält. Außerdem steigert die Schneidkante 33 die Dichtigkeit zu der Oberwand 14, so daß der gesamte Umfangsbereich des Hohlraumes 15 zwischen der Unterfläche 14d der Oberwand 14 und der Innenfläche 19 des Bodens 12 des Hohlraumes abgeschirmt ist und bei Perforation der Oberwand 14 ausgestanzte Partikel nicht zu der Auslaßöffnung 29 gelangen können. Die Partikel sammeln sich in der Höhlung 36 des Filterkörpers 30 und verstopfen weder die Auslaßöffnung 29 noch den Katheter 26.

Der Bodenteil 32 des Filterkörpers 30 hat zusätzlich zu seiner Trennfunktion die Aufgabe, den Boden 12 der Kapsel 11 gegen das Eindringen der Spitze einer zu kräftig eingestochenen Kanüle zu schützen. Bei Ausbildung des Filterkörpers 30 aus metallischem Sintermaterial bildet der Bodenteil 32 eine Auflage, die für die Kanülenspitze undurchdringlich ist, so daß der Portboden 12 nicht versehentlich perforiert werden kann.

Bei dem Beispiel nach Figur 5 ist ein Filterkörper 130 aus geeignetem Sintermaterial, das eine Porengröße von beispielsweise 20 µm haben kann, ebenfalls aus einem Ring 131 mit einem Bodenteil 132 gebildet. Die Außenwand 134 des Ringes 131 ist kreiszylindrisch gestaltet und die Innenwand 135 verläuft konisch gegen den Bodenteil 132 verjüngt. Der freie Rand 137 dieses Filterkörpers 130 ist flach gestaltet und erstreckt sich parallel zur Innenfläche des Bodenteiles 132. Der freie Rand 137 liegt flach gegen die Unterfläche einer elastomeren Oberwand an, die von einem mit der Umfangswand 113 verklebten oder verschweißten Haltering angepreßt wird. Eine gewisse Abdichtung des Umfangsbereiches des Filterkörpers 130 zu der elastomeren Oberwand wird durch einen scharfen ringförmigen Grat 140 erreicht, der koaxial zum Hohlraum an der Umfangswand 113 der Kapsel 111 ausgebildet ist und in die Unterfläche der Oberwand eindrückt. Ein äußerer radialer Flansch 121 sowie ein Anschlußstutzen 123 ergänzen den Port 110 gemäß Figur 5. Die in den Anschlußstutzen 123 eingesetzten Teile 124, 125 und 126 sind mit den entsprechenden Teilen des Beispieles der Figuren 1 und 2 identisch.

Wenn die Höhe des Hohlraumes einer Kapsel 11 oder 111 eines Ports 10 oder 110 nicht ausreicht, um einen Ring eines Filterkörpers mit einem Bodenteil auszustatten, kann auf diesen verzichtet werden und es ergeben sich Formen gemäß Figuren 6 bis 9. Der Filterkörper 230 ist lediglich als Ring 231 mit kreiszylindrischer Außenwand 234 und konischer Innenwand 235 ausgebildet, der an seinem der Oberwand des Ports zugekehrten Ende eine Schneidkante 233 aufweist, die in die Unterfläche der Oberwand eindringt und die in Verbindung mit dem Beispiel der Figuren 1 und 2 beschriebenen Vorteile bewirkt. Der untere Rand 237 des Ringes 231 ist stumpf und eben, so daß er planliegend gegen die Innenfläche 19 des Bodens 12 der Kapsel 11 anliegt.

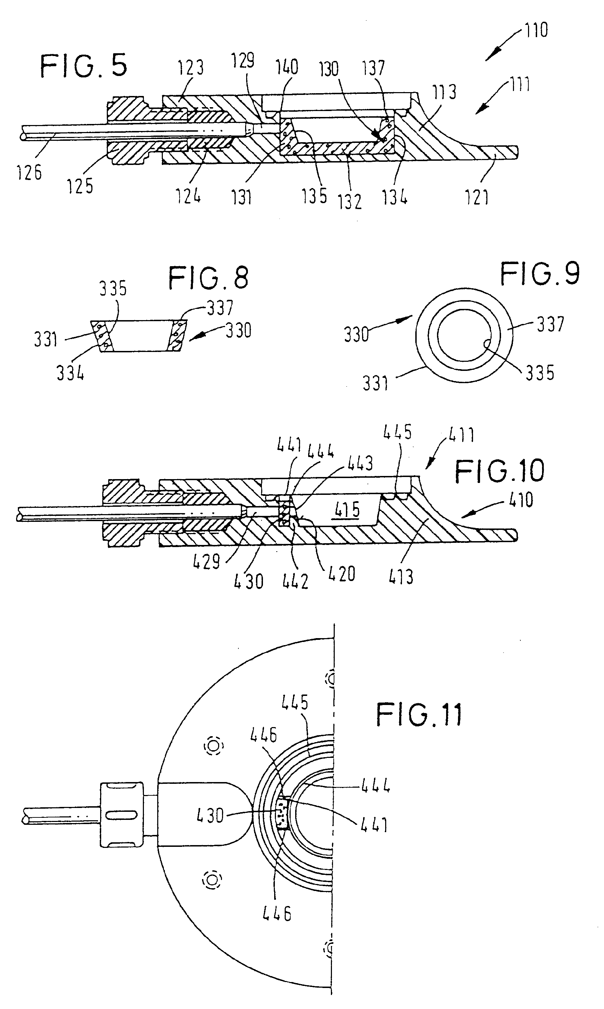

Auch das Beispiel der Figuren 8 und 9 zeigt einen Filterkörper 330 aus hochporösem Sintermaterial, der nur aus einem an beiden Enden offenen Ring 331 besteht. Die Außenwand 334 dieses Ringes 331 und seine Innenwand 335 sind zueinander parallel und konisch. Dieser Filterkörper 330 paßt in einen Hohlraum mit konischer Innenseite, dessen Höhe so bemessen ist, daß der flache obere Rand 337 fest gegen die Unterfläche der elastomeren Oberwand angepreßt ist.

Das Beispiel der Figuren 10 und 11 zeigt eine allgemeine Form der Ausbildung eines Filterkörpers 430 aus hochporösem Sintermaterial. Dieser Filterkörper 430 bildet eine nur leicht gewölbte parallelflächige Scheibe, die der Auslaßöffnung 429 in der Umfangswand 413 einer Kapsel 411 auf der Innenseite vorgesetzt ist. Die Scheibe sitzt in einer Tasche 441, die an der inneren Mündung der Auslaßöffnung 429 gebildet ist und gegen den Hohlraum 415 der Kapsel 411 durch eine die Innenseite 420 des Hohlraumes 415 bildende Innenwand 442 begrenzt ist. Zur Verhinderung der umfangsmäßigen Verschiebung der kurzen Scheibe des Filterkörpers 430 ist die Tasche 441 durch Seitenteile 446 verschlossen. Die Innenwand 442 weist eine Bohrung 443 auf, durch die flüssiges Medikament aus dem Hohlraum 415 zu der Auslaßöffnung 429 gelangt, wobei es von dem Filterkörper 430 zur Absonderung von Hobelspänen aus der elastomeren Oberwand gefiltert wird. Die Innenwand 442 ist an ihrem oberen Rand mit einer scharfen ringförmigen Kante 444 versehen, die sich rings um dem Hohlraum 415 erstreckt und der Abdichtung durch Eindringung in die Unterfläche der Oberwand dient. Eine parallele scharfe ringförmige Kante 445 auf der äußeren Seite des Filterkörpers 430 kann den Abdichteffekt erhöhen. Die übrigen Bestandteile des Ports 410 entsprechen den mit Bezug auf Figuren 1 und 2 beschriebenen Teilen.

## Patentansprüche

1. Port zur Einspritzung von Medikamenten durch einen implantierten katheter (26), bestehend aus einer Kapsel (11), die aus einem Boden (12), einer einen Hohlraum (15) umgebenden Umfangswand (13) und einer den Hohlraum (15) verschließenden durchstechbaren Oberwand (14) gebildet ist und die in der Umfangswand (13) eine Auslaßöffnung (29) aufweist, die innen durch einen gegen den Querschnitt der Auslaßöffnung (29) in der Umfangswand (13) angesetzten Filterkörper abgeschirmt ist und an die ein Ende des Katheters (26) angeschlossen ist,
**dadurch gekennzeichnet,** daß der Filterkörper (30;130;230;330) als topf- oder ringförmiges Einsatzteil (31;131;231;331) aus hochporösem Sintermaterial ausgebildet ist, dessen Umfangsform und Höhe dem Hohlraum der Kapsel angepaßt ist.

2. Port zur Einspritzung von Medikamenten durch einen implantierten katheter (26), bestehend aus einer Kapsel (11), die aus einem Boden (12), einer einen Hohlraum (15) umgebenden Umfangswand (13) und einer den Hohlraum (15) verschließenden durchstechbaren Oberwand (14) gebildet ist und die in der Umfangswand (13) eine Auslaßöffnung (29) aufweist, die innen durch einen gegen den Querschnitt der Auslaßöffnung (29) in der Umfangswand (13) angesetzten Filterkörper abgeschirmt ist und an die ein Ende des Katheters (26) angeschlossen ist,
**dadurch gekennzeichnet,** daß der Filterkörper (430) als Scheibe aus hochporösem Sintermaterial ausgebildet ist, die in einer oben offenen Tasche (441) der Umfangswand (413) angeordnet ist, deren Innenwand (442) eine Bohrung (443) aufweist, die mit der Auslaßöffnung (429) fluchtet.

3. Port nach Anspruch 1,
**dadurch gekennzeichnet,** daß der topf- oder ringförmige Einsatz (31;131;231;331) außen und/oder innen konisch bzw. zylindrisch ist.

4. Port nach Anspruch 1 oder 3,
**dadurch gekennzeichnet,** daß der Bodenteil (32;132) des topfformigen Einsatzteils (31,131) gegen den Boden des Hohlraumes der Kapsel (11;111) anliegt.

5. Port nach einem der Ansprüche 1, 3 oder 4,
**dadurch gekennzeichnet,** daß der der Oberwand benachbarte Rand des Einsatzteils (31;231) als Schneidkante (33;233) ausgebildet ist, die in die Oberwand eindringt.

6. Port nach einem der Ansprüche 1 bis 5,
**dadurch gekennzeichnet,** daß das Sintermaterial aus metallischem Werkstoff, wie rostfreiem Stahl, Nickel, Hastelloy C, B und X, Titan, Monel, Inconel, Incolog besteht.

7. Port nach einem der Ansprüche 1 bis 5,
**dadurch gekennzeichnet,** daß das Sintermaterial aus Kunststoff besteht.

8. Port nach einem der Ansprüche 1 bis 5,
**dadurch gekennzeichnet,** daß das Sintermaterial aus keramischem Material, wie Glas oder dergleichen, besteht.

## Claims

1. A port for injecting medicaments through an implanted catheter (26), comprising a capsule (11) which is formed by a bottom (12), a circumferential wall (13) enclosing a cavity (15), and a pierceable upper wall (14) closing the cavity (15), and which in the circumferential wall (13) is provided with an outlet opening (29) having its interior shielded by a filter member set against the cross-section of the outlet opening (29) in the circumferential wall (13), and having one end of the catheter (26) connected thereto,
**characterized in** that the filter member (30;130;230;330) is formed as a cup-shaped or annular insert (31;131;231;331) of a highly porous sintered material having its circumferential shape and its height adapted to the cavity of the capsule.

2. A port for injecting medicaments through an implanted catheter (26), comprising a capsule (11) which is formed by a bottom (12), a circumferential wall (13) enclosing a cavity (15), and a pierceable upper wall (14) closing the cavity (15), and which in the circumferential wall (13) is provided with an outlet opening (29) having its interior shielded by a filter member set against the cross-section of the outlet opening (29) in the circumferential wall (13), and having one end of the catheter (26) connected thereto,
**characterized in** that the filter member (430) is provided as a disk of a highly porous sintered material which is arranged in an upwardly open pocket (441) of the circumferential wall (413), the inner wall (442) of said pocket (441) having a bore (443) formed therein alignment with the outlet opening (429).

3. The port according to claim 1, characterized in that the cup-shaped or annular insert (31;131;231; 331) is conical or cylindrical on the outside and/ or the inside.

4. The port according to claim 1 or 3, characterized in that the bottom portion (32;132) of the cup-shaped insert (31; 131) abuts on the bottom of the cavity of the capsule (11; 111).

5. The port according to any one of claims 1, 3 or 4, characterized in that the rim of the insert (31; 231) adjacent the upper wall is formed as a cutting edge (33;233) penetrating into the upper wall.

6. The port according to any one of claims 1 to 5, characterized in that the sintered material comprises a metallic material such as stainless steel, nickel, Hastelloy C, B and X, titanium, Monel, Inconel, Incoloy.

7. The port according to any one of claims 1 to 5, characterized in that the sintered material comprises plastic.

8. The port according to any one of claims 1 to 5, characterized in that the sintered material comprises a ceramic material such as glass or the like.

## Revendications

1. Dispositif d'admission pour injection de médicaments à travers un cathéter implanté (26), constitué d'une capsule (11), qui est formée d'un fond (12), d'une paroi périphérique (13) entourant un espace creux (15) et d'une paroi supérieure (14) perforable obturant l'espace creux (15) et qui présente dans la paroi périphérique (13) une ouverture d'évacuation (29), qui est protégée à l'intérieur par un corps filtrant appliqué contre la section transversale de l'ouverture d'évacuation (29) dans la paroi périphérique (13) et à laquelle une extrémité du cathéter (26) est raccordée, caractérisé en ce que le corps filtrant (30, 130, 230, 330) se présente sous la forme d'une pièce insérée en forme de pot ou d'anneau (31, 131, 231, 331) constituée de matière frittée très poreuse, dont la forme périphérique et la hauteur correspondent à l'espace creux de la capsule.

2. Dispositif d'admission pour injection de médicaments à travers un cathéter implanté (26), constitué d'une capsule (11), qui est formée d'un fond (12), d'une paroi périphérique (13) entourant un espace creux (15) et d'une paroi supérieure (14) perforable obturant l'espace creux (15) et qui présente dans la paroi périphérique (13) une ouverture d'évacuation (29), qui est protégée à l'intérieur par un corps filtrant appliqué contre la section transversale de l'ouverture d'évacuation (29) dans la paroi périphérique (13) et à laquelle une extrémité du cathéter (26) est raccordée, caractérisé en ce que le corps filtrant (430) se présente sous la forme d'un disque constitué de matière frittée très poreuse, qui est agencé dans une poche (441) de la paroi périphérique (413) ouverte en haut, dont la paroi interne (442) présente un alésage (443) qui s'aligne avec l'ouverture d'évacuation (429).

3. Dispositif d'admission selon la revendication 1, caractérisé en ce que la pièce insérée en forme de pot ou d'anneau (31, 131, 231, 331) est conique vers l'extérieur et/ou l'intérieur ou bien cylindrique.

4. Dispositif d'admission selon la revendication 1 ou 3, caractérisé en ce que la partie de fond (32, 132) de la pièce insérée en forme de pot (31, 131) s'applique contre le fond de l'espace creux de la capsule (11, 111).

5. Dispositif d'admission selon la revendication 1, 3 ou 4, caractérisé en ce que le bord de la pièce insérée (31, 231) voisin de la paroi supérieure se présente sous la forme d'une arête coupante (33, 233) qui pénètre dans la paroi supérieure.

6. Dispositif d'admission selon l'une quelconque des revendications 1 à 5, caractérisé en ce que la matière frittée est constituée d'un matériau métallique tel que l'acier inoxydable, le nickel, le hastelloy C, B et X, le titane, le monel, l'inconel et l'incoloy.

7. Dispositif d'admission selon l'une quelconque des revendications 1 à 5, caractérisé en ce que la matière frittée est constituée d'une matière synthétique.

8. Dispositif d'admission selon l'une quelconque des revendications 1 à 5, caractérisé en ce que la matière frittée est constituée d'une matière céramique telle que le verre ou une matière analogue.
